# EUROPÄISCHE PATENTANMELDUNG

(11) **EP 1 759 635 A1**
(43) Veröffentlichungstag der Anmeldung: **07.03.2007**
(21) Anmeldenummer: 05019190.7
(22) Anmeldetag: 03.09.2005
(51) Int. Cl.: A61B 5/15

(54) **Verfahren zum Erzeugen einer Einstichwunde und dafür geeignetes Handgerät**

(71) Anmelder: Roche Diagnostics GmbH, 68305 Mannheim (DE); F.HOFFMANN-LA ROCHE AG, 4070 Basel (CH)
(72) Erfinder: Hein, Dr. Heinz-Michael, 64293 Darmstadt (DE); Calasso, Dr. Irio, 6415 Arth (CH); List, Hans, 64754 Hesseneck-Kailbach (DE)
(74) Vertreter: Pfeifer, Hans-Peter

(57) **Zusammenfassung**

Die Erfindung betrifft ein Verfahren zum Erzeugen einer Einstichwunde zum Gewinnen einer Probe einer Körperflüssigkeit aus einem Körperteil, bei welchem in einem Hautöffnungsschritt an einer Einstichstelle in der Epidermis (20) eine Hautöffnung (21) erzeugt wird, danach in einem Probengewinnungsschritt mittels eines Stechelementes (11) ein Probengewinnungsstich ausgeführt wird, bei dem mit dem Stechelement (11) die Hautöffnung (21) vertieft und so eine Einstichwunde zum Gewinnen der Probe erzeugt wird. Die Erfindung betrifft ferner ein Handgerät, mit dem dieses Verfahren durchführbar ist.

## Beschreibung

Die Erfindung betrifft ein Verfahren zum Erzeugen einer Einstichwunde zum Gewinnen einer Probe einer Körperflüssigkeit aus einem Körperteil, sowie ein dafür geeignetes Handgerät umfassend ein Stechelement, einen Antrieb, mit dem das Stechelement in Richtung auf die Haut beweglich und in Richtung von der Haut weg beweglich ist, und eine Steuereinrichtung zum Steuern der Bewegung des Stechelementes.

Um für analytisch-diagnostische Zwecke eine geringe Menge Blut oder interstitielle Flüssigkeit aus einem Körperteil, beispielsweise dem Finger, zu entnehmen, werden Stechelemente, beispielsweise Nadeln oder Lanzetten, verwendet, die zur Erzeugung einer Einstichwunde in das entsprechende Körperteil gestochen werden. Soweit dies manuell geschieht ist speziell trainiertes Personal erforderlich. Dennoch ist der Einstich mit erheblichem Schmerz verbunden.

Bereits seit langem werden Blutentnahmesysteme verwendet, die aus einem Stechgerät und zugehörigen, für das jeweilige Gerät speziell angepaßten Lanzetten bestehen. In einem Gehäuse des Stechgeräts befindet sich ein Antrieb, mit dem das Stechelement in Richtung auf die Haut beweglich und in Richtung von der Haut weg beweglich ist. Als Antriebselement für die Einstichbewegung dient eine Feder. Zu Beginn der Entwicklung waren sehr einfache Konstruktionen gebräuchlich, bei denen die Lanzette unmittelbar an einem Ende einer in einem länglichen Gehäuse angeordneten Druckfeder befestigt war (z.B. US-Patent 4,469,110).

Derartige Blutentnahmesysteme wurden jedoch den hohen Anforderungen nicht gerecht, die zu erfüllen sind, wenn eine regelmäßige Überwachung analytischer Werte des Blutes erforderlich ist. Dies gilt insbesondere für Diabetiker, die ihren Blutzuckerspiegel häufig kontrollieren sollten, um ihn durch Insulininjektion innerhalb bestimmter Sollgrenzen halten zu können. Durch umfangreiche wissenschaftliche Untersuchungen wurde bewiesen, daß mittels einer Intensivtherapie mit mindestens vier Blutanalysen pro Tag eine dramatische Reduzierung schwerster Spätschäden des Diabetis mellitus (beispielsweise eine Retinopathie mit resultierender Erblindung des Patienten) erreicht werden kann.

Diese Intensivtherapie setzt voraus, daß die Blutentnahme mit einem möglichst geringen Schmerz verbunden ist. Mit dem Ziel, diesbezüglich eine Verbesserung zu erreichen, wurden zahlreiche unterschiedliche Blutentnahmesysteme entwickelt.

Als wesentlich für eine möglichst schmerzarme Blutentnahme wird es angesehen, daß die Einstich- und Rückführbewegung des Stechelementes möglichst schnell, vibrationsfrei und mit einer optimalen Einstichtiefe erfolgt. Als optimal wird dabei eine Einstichtiefe angesehen, die nicht größer ist, als zum Erreichen blutführender Gewebeschichten unbedingt erforderlich.

Eine relativ schmerzarme Blutentnahme ermöglichen Handgeräte des in der US 2004/0092996 A1 beschriebenen Typs. Bei derartigen Handgeräten umfaßt der Antrieb eine Antriebsfeder zum Erzeugen einer Antriebskraft und einen Antriebsrotor, der unter Einwirkung der Antriebskraft eine Drehbewegung ausführt. Drehbewegungen des Antriebsrotors werden von einer Steuereinrichtung, die eine mit dem Antriebsrotor gekoppelte Kurvensteuerung umfaßt, in eine Einstich- und Rückführbewegung des Stechelementes umgesetzt.

Ferner sind beispielsweise aus der EP 1 101 443 B1 elektrische Stechgeräte bekannt, bei denen eine Einstich- und Rückführbewegung der Lanzette durch die Magnetkraft einer Spule bewirkt werden. Elektrische Stechgeräte haben den Vorteil, daß sich die Geschwindigkeit des Stechelementes mit hoher Präzision steuern läßt. In der WO 03/088824 wird diesbezüglich empfohlen, bei einer Einstichbewegung das Stratum corneum mit maximaler Geschwindigkeit zu durchstoßen und die Lanzette anschließend abzubremsen, so daß das Eindringen in tiefere Hautschichten mit geringerer Geschwindigkeit erfolgt. Durch eine derartige Einstichbewegung, bei der die Lanzettengeschwindigkeit mit der Eindringtiefe abnimmt, wird eine Reduktion schmerzhafter Druckwellen angestrebt.

Trotz der umfangreichen Entwicklungsarbeiten, die zu den vorstehend erörterten und zahlreichen weiteren Konstruktionen geführt haben, besteht nach wie vor ein großes Interesse an Probengewinnungssystemen und -verfahren, welche die schwierigen und teilweise gegenläufigen Anforderungen (minimales Schmerzempfinden, zuverlässige Gewinnung einer ausreichenden Probenmenge, einfache Bedienbarkeit, kompakte Bauweise, kostengünstige Konstruktion) gleichzeitig möglichst weitgehend erfüllen.

Aufgabe der Erfindung ist es insbesondere, einen Weg aufzuzeigen, wie eine Einstichwunde zum Gewinnen einer Probe einer Körperflüssigkeit aus einem Körperteil mit noch geringerem Schmerz erzeugt werden kann.

Diese Aufgabe wird gelöst durch ein Verfahren zum Erzeugen einer Einstichwunde zum Gewinnen einer Probe einer Körperflüssigkeit aus einem Körperteil, bei welchem in einem Hautöffnungsschritt an einer Einstichstelle in der Epidermis eine Hautöffnung erzeugt wird, und danach in einem Probengewinnungsschritt mittels eines Stechelementes ein Probengewinnungsstich ausgeführt wird, bei dem mit dem Stechelement die Hautöffnung vertieft und so eine Einstichwunde zum Gewinnen der Probe erzeugt wird.

Die Aufgabe wird ferner gelöst durch ein Handgerät zum Erzeugen einer Einstichwunde umfassend ein Stechelement, einen Antrieb, mit dem das Stechelement in Richtung auf die Haut hin beweglich und in Richtung von der Haut weg beweglich ist, und eine Steuereinrichtung zum Steuern der Bewegung des Stechelementes, dadurch gekennzeichnet, daß es Mittel aufweist, durch die in einem Hautöffnungsschritt an einer Einstichstelle in der Epidermis eine Hautöffnung erzeugt wird, und die Steuereinrichtung so eingerichtet ist, daß nach dem Hautöffnungsschritt in einem Probengewinnungsschritt mittels eines Stechelementes ein Probengewinnungsstich ausgeführt wird, bei dem mit dem Stechelement die Hautöffnung vertieft und so eine Einstichwunde zum Gewinnen der Probe erzeugt wird.

Während nach dem Stand der Technik eine Einstichwunde in einem einzigen Schritt mit einer Einstich- und Rückführbewegung erzeugt wird, werden erfindungsgemäß zwei getrennte Schritte ausgeführt, nämlich zunächst ein Hautöffnungsschritt, in dem an einer Einstichstelle in der Epidermis eine Hautöffnung erzeugt wird, und danach ein Probengewinnungsschritt, in dem mittels eines Stechelementes ein Probengewinnungsstich ausgeführt wird, bei dem die Hautöffnung vertieft und so eine Einstichwunde erzeugt wird.

Eine wesentliche Grundlage der Erfindung ist die Erkenntnis, daß bei einer herkömmlichen Einstich- und Rückführbewegung das Stechelement durch Reibung an oberen Hautschichten, insbesondere dem Stratum corneum, abgebremst wird, bevor tiefere, blutgebende Gewebeschichten erreicht werden. Dadurch entsteht eine Druckwelle, die sich ausgehend von Reibungsflächen der Lanzette durch das Gewebe ausbreitet und Schmerz verursacht. Das Entstehen einer schmerzhaften Druckwelle kann vermieden werden, indem zunächst in einem Hautöffnungsschritt an einer Einstichstelle in der Epidermis eine Hautöffnung erzeugt wird, deren Tiefe so gering ist, daß dabei praktisch kein Schmerz entsteht. Das Stratum corneum und tieferliegende Schichten der Epidermis können innerhalb kürzester Zeit relaxieren, so daß bei einem später erfolgendem Probengewinnungsschritt, beispielsweise 1 msec nach dem Ende des Hautöffnungsschritts, die Hautöffnung vertieft und eine Einstichwunde zum Gewinnen der Probe erzeugt werden kann. Auch dabei ist der Schmerz geringer als bei bekannten Verfahren. Bei optimaler Einstellung der Stichparameter ist er nur unwesentlich größer als bei dem Hautöffnungsschritt, so daß insgesamt eine weitgehend schmerzfreie Blutgewinnung erzielt wird. Da bei dem Probengewinnungsstich bereits eine Hautöffnung vorhanden ist, ist die auftretende Reibung so gering, daß eine schmerzhafte Druckwelle vermieden wird.

Der Hautöffnungsschritt kann beispielsweise dadurch von dem Probengewinnungsschritt getrennt sein, daß für die Hautöffnung ein anderes Mittel als das Stechelement verwendet wird. Geeignet ist beispielsweise ein Laser, durch den ein kurzer Laserlichtpuls zum Verdampfen eines Teils der Epidermis an der Einstichstelle, beispielsweise eines großen Teils des Stratum corneums erzeugt wird. Bevorzugt wird für den Hautöffnungsschritt jedoch das selbe Stechelement wie für den Probengewinnungsschritt verwendet. Dabei kann der Probengewinnungsschritt von dem Hautöffnungsschritt dadurch abgetrennt sein, daß das Stechelement am Ende des Hautöffnungsschritts abgebremst, vorzugsweise gestoppt, und für den Probengewinnungsschritt erneut beschleunigt wird. Wenn sich das Stechelement nach dem Abbremsen nur langsam oder gar nicht in Einstichrichtung bewegt, kann die Epidermis relaxieren, bevor die Hautöffnung durch den Probengewinnungsschritt vertieft wird.

Ein weiterer wichtiger Vorteil der Erfindung besteht darin, daß sich die Einstichtiefe wesentlich präziser einstellen und kontrollieren läßt, als dies bei Verfahren nach dem Stand der Technik der Fall ist. Bei einer herkömmlichen Einstich- und Rückführbewegung nach dem Stand der Technik führen Reibungskräfte zu einer erheblichen Verformung der Hautoberfläche. Beim Durchstoßen des Stratum corneums entsteht eine Vertiefung (Delle) der Haut an der Einstichstelle, so daß die Lanzette weniger tief unter die deformierte Hautoberfläche vordringt, als aufgrund des Lanzettenhubs beim Ansetzen des Gerätes an die Hautoberfläche an sich zu erwarten wäre.

Die Distanz, um die die Epidermis und blutführende Gewebeschichten durch diesen Effekt vor dem Stechelement zurückweichen, hängt von der Kinetik der Einstichbewegung, der Form des Stechelementes und nicht zuletzt den elastischen Eigenschaften der Haut des Patienten zum Zeitpunkt des Einstichs an der Einstichstelle ab. Wird vor dem Probengewinnungsschritt zunächst in einem Hautöffnungsschritt an der Einstichstelle eine Hautöffnung in der Epidermis erzeugt, tritt der Effekt des Eindellens der Haut an der Einstichstelle nicht mehr oder nur noch so stark reduziert auf, daß die Einstichtiefe wesentlich präziser kontrolliert und eingestellt werden kann. Durch die Erfindung wird es deshalb möglich, einen Einstich nur so tief auszuführen, wie es zum Gewinnen der Probe unbedingt nötig ist.

Bevorzugt liegt die bei dem Hautöffnungsschritt erzeugte Hautöffnung vollständig in dem Stratum corneum. Es ist jedoch akzeptabel, wenn das Stechelement bei dem Hautöffnungsschritt auch in eine tieferliegende Schicht der Epidermis, also das Stratum lucidum, Stratum granulosum, Stratum spinosum oder sogar in das Stratum basale eindringt, da auch diese Schichten weder Nerven noch Blutgefäße enthalten. Jedenfalls sollte sichergestellt sein, daß erst in dem Probengewinnungsschritt blutgebendes Gewebe erreicht wird. In der Regel wird ein praktisch schmerzfreier Hautöffnungsschritt dadurch erreicht, daß die bei dem Hautöffnungsschritt erzeugte Hautöffnung eine Tiefe von mindestens 0,8 mm und höchstens 1,2 mm hat.

Bevorzugt wird das Stechelement nach dem Hautöffnungsschritt abgestoppt und zurückgezogen. Besonders bevorzugt wird es vollständig aus der Epidermis herausgezogen, so daß bei dem Probengewinnungsstich des Probengewinnungsschritts ein erneuter Einstich erfolgt. Wie bereits erwähnt, ist es jedoch prinzipiell ausreichend, daß das Stechelement am Ende des Hautöffnungsschritts so stark und so lange abgebremst wird, daß die Epidermis relaxieren kann, bevor das Stechelement erneut beschleunigt und die Hautöffnung durch Vorschieben des Stechelementes zum Erzeugen der Einstichwunde vertieft wird. Das Zurückziehen, insbesondere das vollständige Zurückziehen ermöglicht es, das Stechelement vor seinem Eindringen in blutführende Gewebeschichten auf eine höhere Geschwindigkeit zu beschleunigen, so daß der Probengewinnungsstich besonders schnell und damit besonders schmerzarm ausgeführt werden kann. Hinzu kommt, daß bei dem Zurückziehen des Stechelements der Relaxationsprozeß durch Reibungskräfte unterstützt wird, so daß der Probengewinnungsstich praktisch sofort nach dem vollständigen Herausziehen des Stechelements der Haut durchgeführt werden kann.

In anderem Zusammenhang ist es aus der WO2004/041087 bekannt, mit einem Stechelement hintereinander zweimal in dieselbe Einstichstelle einzustechen. Im Unterschied zu der vorliegenden Erfindung wird dabei jedoch bereits mit dem ersten Stich eine blutgebende Gewebeschicht erreicht. Der zweite Stich wird mit einer geringeren Tiefe ausgeführt. Bei der vorliegenden Erfindung sind die geometrischen Verhältnisse umgekehrt, da erfindungsgemäß bei dem zweiten Stich, nämlich dem Probengewinnungsstich, eine größere Tiefe als bei dem ersten Stich, dem Hautöffnungsstich, erreicht wird. Das bekannte Verfahren hat im übrigen auch nicht das Ziel, eine Reduktion des mit der Probengewinnung verbundenen Schmerzes zu erreichen, sondern die aus der Einstichwunde austretende Blutmenge zu erhöhen. Der zweite Stich dient vielmehr dazu, ein vorzeitiges Verschließen der Einstichwunde zu verhindern, wobei Blut durch einen Kapillarspalt des Einstichelementes aus der Einstichwunde gefördert wird. Diese Erkenntnis der WO 2004/041087 kann bei der vorliegenden Erfindung durch einen dritten Stich genutzt werden, der eine geringe Einstichtiefe als der Probengewinnungsstich hat.

Die Erfindung wird nachfolgend anhand von Ausführungsbeispielen von Handgeräten zur Durchführung des beschriebenen Verfahrens unter Bezugnahme auf die beigefügten Zeichnungen näher erläutert. Die darin dargestellten Besonderheiten können einzeln oder in Kombination verwendet werden, um bevorzugte Ausgestaltungen zu schaffen. Gleiche oder aneinander entsprechende Bauteile sind mit übereinstimmenden Bezugszeichen gekennzeichnet. Es zeigen:
- Fig. 1: ein erfindungsgemäßes Handgerät;
- Fig. 2: einen Spulenkörper des Stechelementantriebs des in Figur 1 gezeigten Gerätes;
- Fig. 3: den Antrieb des in Figur 1 gezeigten Gerätes in einer Seitenansicht;
- Fig. 4: ein Stechprofil eines erfindungsgemäßen Verfahrens;
- Fig. 5: eine schematische Schnittansicht einer Geräteöffnung mit daran anliegender Hautoberfläche;
- Fig. 6: eine Darstellung gemäß Figur 5 bei einem Hautöffnungsstich;
- Fig. 7: eine Darstellung gemäß Figur 5 mit einer in der Hautoberfläche erzeugten Hautöffnung;
- Fig. 8: eine Darstellung gemäß Figur 5 während des Vertiefens der Hautoberfläche zum Erzeugen einer Einstichwunde;
- Fig. 9: eine Steuerkurve eines Handgeräts mit einem Rotorantrieb.

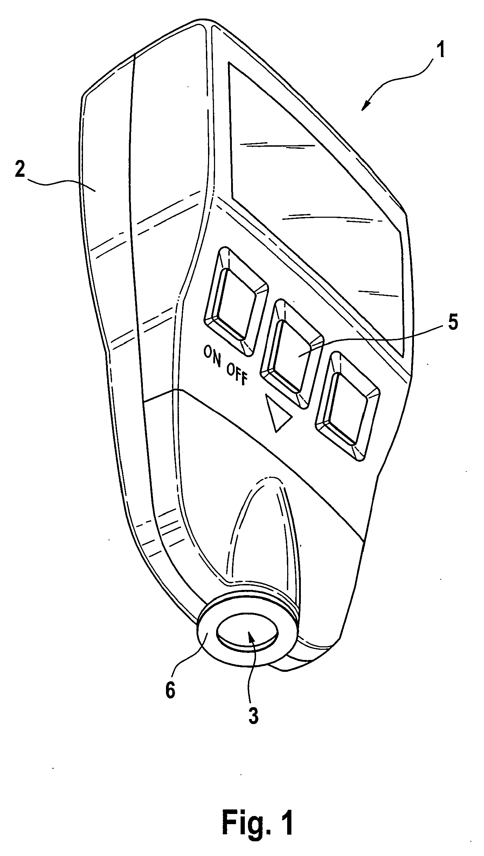

Das in Figur 1 gezeigte Handgerät 1 dient zum Erzeugen einer Einstichwunde zum Entnehmen von Körperflüssigkeit, insbesondere Blut und/oder interstitieller Flüssigkeit, für Diagnosezwecke. Das Gehäuse 2 weist eine Gehäuseöffnung 3, an die bestimmungsgemäß ein Finger angelegt wird, und Bedingungselement 5 in Form von Tasten auf.

Um die Durchblutung des Gewebes an der Einstichstelle zu fördern, ist es günstig, wenn die Gehäuseöffnung 3 von einem Andruckring 6 umgeben ist, der sich beim Andrücken eines Körperteils elastisch deformiert. Beispielsweise kann der Andruckring 6 aus einem gummielastischem Kunststoff hergestellt sein. Bevorzugt hat der Andruckring 6 eine nach innen geneigte Andruckfläche, an die bestimmungsgemäß ein Finger oder ein anderes Körperteil angelegt wird. Ein geeigneter Andruckring ist detailliert in der WO 01/89 383 A2 beschrieben, die diesbezüglich durch Bezugnahme zum Gegenstand der Anmeldung gemacht wird. In der WO 01/89 383 A2 wird der Andruckring als Kompressionseinheit bezeichnet.

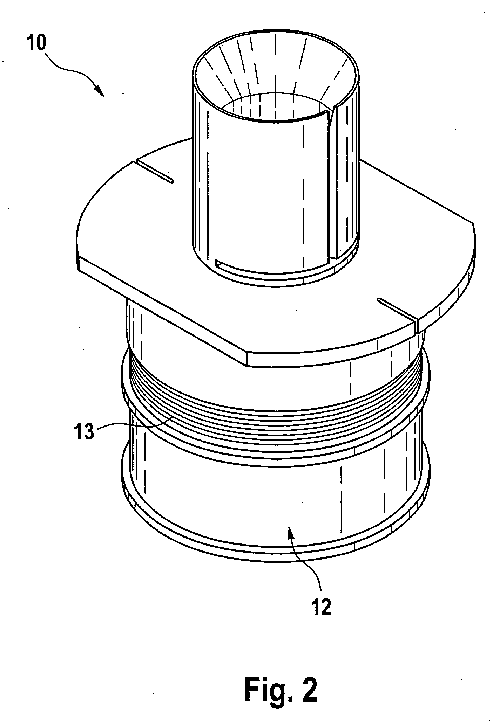

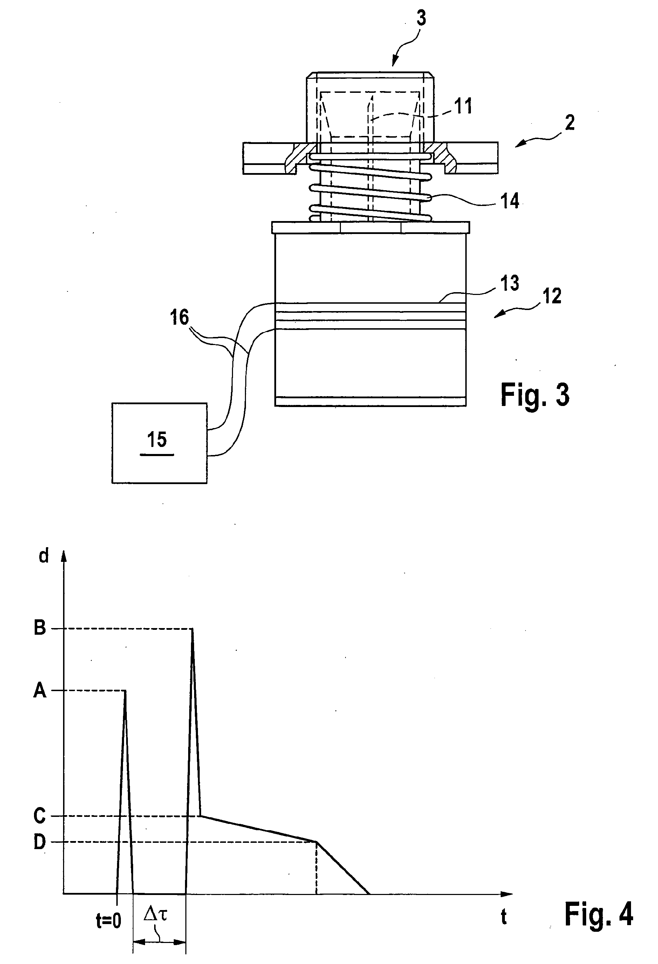

Das Handgerät 1 hat einen Antrieb 10, dessen wesentliche Bauteile in den Figuren 2 und 3 dargestellt sind und mit dem das in Fig. 3 dargestellte Stechelement 11 in Richtung auf die Haut hin beweglich und in Richtung von der Haut weg beweglich ist. Bei dem Antrieb 10 handelt es sich um einen elektromagnetischen Antrieb, wie er beispielsweise in der EP 1 101 443 B1 beschrieben ist, die hinsichtlich der Ausgestaltung des Antriebs durch Bezugnahme zum Gegenstand der vorliegenden Anmeldung gemacht wird.

Der Antrieb 10 umfaßt einen Spulenkörper 12 aus Kunststoff, der eine Spule 13 trägt. Der Spulenkörper 12 ist von einer ortsfesten Magnethülse (nicht gezeigt) umgeben.

Fließt durch die Spule 13 ein elektrischer Strom, so entsteht eine magnetische Kraft, deren Richtung und Stärke von der Richtung und Stärke des elektrischen Stroms abhängt. Je nach Richtung der magnetischen Kraft wird der Spulenkörper 12 für eine Einstichbewegung aus der Magnethülse heraus, d.h. vorwärts, geschoben oder für eine Rückführbewegung zurückgezogen. Zur Steuerung der Stromstärke und damit der Bewegung des Stechelementes 11 dient eine Steuereinrichtung 15 mit Mikroprozessorsteuerung, die über Anschlußleitungen 16 an die Spule 13 angeschlossen ist.

Wie Figur 3 zeigt, trägt der Spulenkörper 12 ein Stechelement 11 in Form einer Lanzette. Damit das Stechelement 11 nur dann aus der Geräteöffnung 3 herausragt, wenn durch einen entsprechenden Spulenstrom eine den Spulenkörper 12 nach vorn schiebende Magnetkraft vorhanden ist, ist zwischen dem Spulenkörper 12 und dem Gehäuse 2 eine Kompressionsfeder 14 angeordnet. Wird der Spulenkörper 12 nach vorne geschoben, führt dies zu einer Kompression der Feder 14 und einer entsprechenden Rückstellkraft.

Zum Gewinnen einer Probe einer Körperflüssigkeit aus einem an die Gehäuseöffnung 3 angelegten Finger wird das Stechelement 11 in einem Hautöffnungsschritt so weit vorgeschoben, daß die Spitze des Stechelementes in die Epidermis eindringt, jedoch keine blutführenden Gewebeschichten erreicht werden. Bei den meisten Menschen ist dafür eine Eindringtiefe von etwa 0,8 mm bis 1,2 mm optimal. Der für einen entsprechenden Lanzettenhub benötigte Spulenstrom wird durch die Steuereinrichtung 15 gesteuert.

Nach dem Hautöffnungsstich wird die Stromrichtung umgekehrt, so daß der Spulenkörper 12 zurückgezogen wird. Anschließend wird die Stromrichtung erneut umgekehrt, so daß der Spulenkörper 12 für den Probengewinnungsstich erneut in Richtung auf das an der Öffnung 3 anliegende Körperteil beschleunigt und in die Haut vorgeschoben wird. Für den beschriebenen Fall, daß das Stechelement am Ende des Probengewinnungsschritts abgestoppt wird, liegt bevorzugt zwischen dem Ende des Hautöffnungsschritts und dem Beginn des Probengewinnungsschritts ein zeitlicher Abstand von 1 msec bis 1 sec, besonders bevorzugt 1 msec bis 30 msec. Dabei wird das Ende des Hautöffnungsschritts durch das Abstoppen des Stechelements bei dem Hautöffnungsstich und der Beginn des Probengewinnungsschritts durch das erneute Beschleunigen des Stechelementes für den Probengewinnungsstich definiert. Zwischen dem Ende des Hautöffnungsschritts und dem Beginn des Probengewinnungsschritts wird bevorzugt das Stechelement zurückgezogen, vorzugsweise vollständig aus der Epidermis herausgezogen.

Bei dem Hautöffnungsstich wird die Hautoberfläche an der Einstichstelle eingedellt, da das Stratum corneum aufgrund seiner Festigkeit der Spitze des Stechelementes 11 einen Widerstand entgegensetzt. Bei dem anschließenden Probengewinnungsschritt tritt dieser Effekt des Eindellens nicht mehr oder nur noch wesentlich schwächer auf. Deshalb wird bei dem Probengewinnungsschritt auch dann eine größere Stichtiefe erreicht, wenn der Bewegungshub des Stechelementes relativ zu der Öffnung 3, an der das Körperteil anliegt, unverändert ist. Bei gleichbleibendem Hub des Stechelementes wird daher die Hautöffnung in dem Probengewinnungsschritt typischerweise um etwa 100 µm bis 500 µm, bevorzugt 100 µm bis 300 µm, vertieft.

Bevorzugt wird als Stechelement eine Mikronadel verwendet, mit der mittels Kapillarkräften eine kleine Menge Blut aus der erzeugten Einstichwunde entnommen wird. Zur Optimierung der Blutgewinnung ist es dabei günstig, das Stechelement nach dem Blutgewinnungsstich um einen Teil der Einstichstrecke in eine Sammelposition zurückzuziehen und dort für eine Sammelperiode von beispielsweise einigen Sekunden verharren zu lassen. Auf diese Weise kann ein Teil des Einstichkanals freigegeben werden, so daß sich Körperflüssigkeit darin sammeln und von dort in eine Kapillarstruktur des Stechelementes eindringen kann.

Ein Beispiel eines Stechprofils, das einen Hautöffnungsstich, einen Probengewinnungsstich und eine anschließende Sammelphase umfaßt, ist in Figur 4 dargestellt. Darin ist die Einstichtiefe d des Stechelementes über der Zeit t aufgetragen. Wie man darin erkennt, dringt das Stechelement bei dem Hautöffnungsstich zunächst bis auf eine Einstichtiefe A ein. Anschließend wird das Stechelement mit einer Rückführbewegung vollständig aus der Haut zurückgezogen. Nach einem Zeitraum Δτ erfolgt der Probengewinnungsstich, bei dem das Stechelement erneut in die erzeugte Hautöffnung eingestochen und die Hautöffnung auf die Einstichtiefe B vertieft wird.

Anschließend wird das Stechelement mit einer Rückführbewegung in eine Sammelposition zurückgezogen, in der es nur noch bis in eine Tiefe C in die Haut hineinragt. Bei Erreichen der Sammelposition wird das Stechelement abgebremst und während einer anschließenden Sammelphase von typischerweise 1 sec bis 3 sec Dauer langsam bis auf eine Tiefe D zurückgezogen. Während der Sammelphase wird über einen Kapillarkanal des Stechelementes Blut aus der Einstichwunde aufgenommen. Nach Abschluß der Sammelphase, d.h. bei Erreichen der Tiefe D, wird das Stechelement vollständig aus der Einstichwunde herausgezogen.

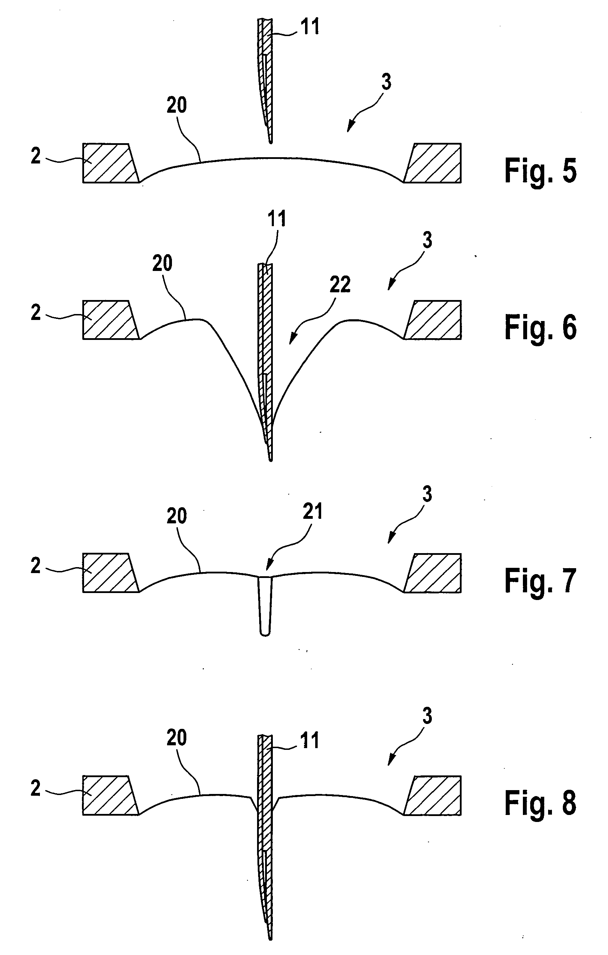

In Figur 5 sind die geometrischen Verhältnisse bei Anlegen eines Körperteils mit einer Hautoberfläche 20 an die Geräteöffnung 3 dargestellt. Durch Anpressen an die Öffnung 3 wölbt sich die Hautoberfläche 20 in die Öffnung hinein. Dies hat eine erhöhte Durchblutung der Einstichstelle zur Folge und erleichtert deshalb die Probengewinnung. Andererseits wird durch die Wölbung der Hautoberfläche 20 eine präzise Reproduzierbarkeit der Einstichtiefe erschwert. Bevorzugt wird deshalb vor dem Hautöffnungsstich die Lage der Hautoberfläche 20 an der Einstichstelle in Relation zu einem festen Referenzpunkt des Handgerätes, beispielsweise dem Gehäuse 2 am Rand der Öffnung 3, bestimmt. Dies kann beispielsweise durch eine optische Messung oder durch eine elektrische Messung, beispielsweise eine Impedanzmessung oder eine kapazitive Messung, geschehen, bei der das Stechelement 11 als Elektrode an die Hautoberfläche 20 herangefahren und anschließend zur Vorbereitung des Hautöffnungsstichs wieder zurückgezogen wird.

Bei dem Hautöffnungsstich setzt das Stratum corneum dem Stechelement 11 einen merklichen Widerstand entgegen. Figur 7 zeigt die dadurch bewirkte Vertiefung (Delle) 22 der Hauoberfläche 20 an der Einstichstelle. Nach dem Hautöffnungsstich wird das Stechelement 11 wieder zurückgezogen, so daß die Hautoberfläche 20 in eine in Figur 7 dargestellte Ruhelage relaxieren kann. Diese Ruhelage ist wegen einer Änderung der elastischen Eigenschaften der Hautoberfläche 20 durch die Hautöffnung 21 nicht mit der in Figur 5 dargestellten Ausgangslage identisch. Im Rahmen der Erfindung konnte jedoch festgestellt werden, daß vorhandene Unterschiede so reproduzierbar auftreten, daß beim Erzeugen der Einstichwunde keine erneute Messung der Position der Hautoberfläche 20 erforderlich ist, sondern die Detektion der Position der Hautoberfläche 20 vor dem Hautöffnungsschritt ausreicht, um eine präzise reproduzierbare Einstichtiefe zu gewährleisten.

Die in Figur 7 dargestellte Hautöffnung 21 wird mit dem in Figur 8 dargestellten Probengewinnungsstich so weit vertieft, daß blutgebendes Gewebe erreicht wird. Wie Figur 8 zeigt, läßt sich dabei die Hautöffnung 21 vertiefen, ohne daß der in Figur 6 dargestellte Effekt des Eindellens der Hautoberfläche 20 in erheblichem Umfang auftritt.

Die beschriebene Erfindung ist insbesondere für Handgeräte mit einer relativ großen Öffnung 3 mit einem Durchmesser von mindestens 3 mm, vorzugsweise mindestens 5 mm geeignet, da sich trotz einer dabei in erheblichem Umfang auftretenden Hautwölbung eine präzise reproduzierbare Einstichtiefe erreichen läßt und folglich die Vorteile einer als Folge einer besseren Durchblutung der Einstichstelle leichteren Probengewinnung genutzt werden können.

Statt des in den Figuren 2 und 3 dargestellten elektromagnetischen Antriebs kann auch ein mechanischer Antrieb verwendet werden, der eine Antriebsfeder zum Erzeugen einer Antriebskraft und einen Antriebsrotor umfaßt, der unter Einwirkung der Antriebskraft eine Drehbewegung ausführt. Die Steuereinrichtung eines derartigen Handgeräts umfaßt eine mit dem Antriebsrotor gekoppelte Kurvensteuerung, durch die Drehbewegungen des Antriebsrotors in Bewegungen des Stechelementes in Richtung auf die Haut hin und in Richtung von der Haut weg umgesetzt werden.

Ein derartiger mechanischer Antrieb ist aus der EP 1 384 438 A1 bekannt und muß deshalb nicht näher erläutert werden. Um das bekannte Gerät so abzuändern, daß in einem Hautöffnungsschritt an einer Einstichstelle in der Epidermis eine Hautöffnung erzeugt wird und danach in einem Probengewinnungsschritt ein Probengewinnungsstich ausgeführt wird, bei dem mit dem Stechelement die Hautöffnung vertieft und so eine Einstichwunde zum Gewinnen der Probe erzeugt wird, genügt es, die Kurvensteuerung so abzuwandeln, daß die Steuerkurve einen Verlauf hat, der ein erstes Maximum zum Erzeugen der Hautöffnung und ein zweites Maximum zum Erzeugen der Einstichwunde aufweist. Bei entsprechend geänderter Anordnung der Kurve in dem Gerät kann alternativ auch eine Steuerkurve mit zwei Minima verwendet werden.

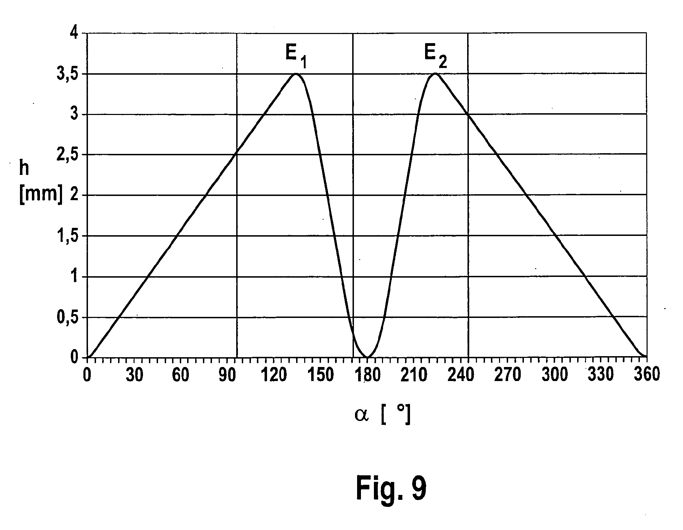

Eine schematische Darstellung einer geeigneten Steuerkurve, die beispielsweise als Nut auf dem Antriebsrotor angeordnet sein kann, zeigt Figur 9. Der Antrieb unterscheidet sich von dem in der EP 1 384 438 A1 beschriebenen Handgerät im wesentlichen nur durch die Form der Steuerkurve. Hinsichtlich der mechanischen Details einer geeigneten Konstruktion wird deshalb auf die EP 1 384 438 A1 verwiesen, die diesbezüglich durch Bezugnahme zum Gegenstand der vorliegenden Anmeldung gemacht wird. Besonders vorteilhaft ist es, in dem Gerät einen Elektromotor zum Spannen der Antriebsfeder anzuordnen. Dieser Elektromotor kann zusätzlich auch andere Aufgaben übernehmen, dient jedoch bevorzugt nur zum Spannen der Antriebsfeder. Zur Energieversorgung des Elektromotors können handelsübliche Batterien, Akkumulatoren oder auch Solarzellen verwendet werden, so daß eine netzunabhängige Stromversorgung des Geräts gegeben ist.

In Figur 9 ist der Lanzettenhub h über der Drehwinkelstellung α des Antriebsrotors in Grad aufgetragen. Bei dem dargestellten Ausführungsbeispiel wird der Lanzettenhub h bei dem Hautöffnungsstich und dem Probengewinnungsstich jeweils durch ein Extremum E₁ bzw. E₂ der Steuerkurve bestimmt und ist im dargestellten Fall für beide Stiche gleich groß. Wie bereits erwähnt, wird bei dem Probengewinnungsstich dennoch eine größere Einstichtiefe erreicht, da sich die Haut bei dem Hautöffnungsschritt merklich eindellt und dieser Effekt des Eindellens bei dem Probengewinnungsstich nicht oder nur wesentlich schwächer auftritt.

Die in Figur 9 dargestellte Steuerkurve umfaßt einen Drehwinkel des Antriebsrotors von 360°. Möglich sind aber auch längere Steuerkurven von mehr als 360°, beispielsweise 540°. Für kompliziertere Bewegungsabläufe, beispielsweise im Zusammenhang mit der beschriebenen Sammelphase, kann es günstig sein, wenn durch Entspannen der Antriebsfeder eine Drehbewegung des Antriebsrotors von mehr als 360°, beispielsweise 540°, bewirkt wird und dabei der Steuerkurvenreiter der Kurvensteuerung einen Drehwinkel der Steuerkurve von mehr als 360°, beispielsweise 540°, abfährt.

Die verwendete Steuerkurve kann eine geschlossene Steuerkurve gemäß Figur 9 sein, so daß diese beim Spannen und Entspannen der Antriebsfeder stets nur in einer Richtung durchlaufen wird. Möglich ist es aber auch, eine offene Steuerkurve, d.h. eine Steuerkurve mit zwei voneinander beabstandeten Enden, zu verwenden, so daß sich der Antriebsrotor beim Entspannen der Antriebsfeder in einer ersten Drehrichtung dreht und der Antriebsrotor zum Spannen der Antriebsfeder in einer zweiten Drehrichtung gedreht wird, die der ersten Drehrichtung entgegengesetzt ist.

### Bezugszeichenliste

- 1: Handgerät
- 2: Gehäuse
- 3: Gehäuseöffnung
- 6: Andruckring
- 10: Antrieb
- 11: Stechelement
- 12: Spulenkörper
- 13: Spule
- 14: Feder
- 15: Seuereinrichtung
- 16: Anschlußleitungen
- 20: Hautoberfläche
- 21: Hautöffnung
- A: Einstichtiefe des Hautöffnungsstichs
- B: Einstichtiefe des Probengewinnungsstichs
- C: Einstichtiefe zu Beginn der Sammelphase
- D: Einstichtiefe am Ende der Sammelphase
- h: Lanzettenhub
- t: Zeit

## Patentansprüche

1. Verfahren zum Erzeugen einer Einstichwunde zum Gewinnen einer Probe einer Körperflüssigkeit aus einem Körperteil, bei welchem
in einem Hautöffnungsschritt an einer Einstichstelle in der Epidermis (20) eine Hautöffnung (21) erzeugt wird, und
danach in einem Probengewinnungsschritt mittels eines Stechelementes (11) ein Probengewinnungsstich ausgeführt wird, bei dem die Hautöffnung mit dem Stechelement (11) vertieft und **dadurch** eine Einstichwunde zum Gewinnen der Probe erzeugt wird.

2. Verfahren nach Anspruch 1, **dadurch gekennzeichnet, daß** zwischen dem Ende des Hautöffnungsschritts und dem Beginn des Probengewinnungsschritts ein zeitlicher Abstand von 1 msec bis 1 sec, vorzugsweise 1 msec bis 30 msec, liegt.

3. Verfahren nach einem der vorhergehenden Ansprüche, **dadurch gekennzeichnet, daß** für den Hautöffnungsschritt ein anderes Mittel als das Stechelement (11) verwendet wird.

4. Verfahren nach einem der vorhergehenden Ansprüche, **dadurch gekennzeichnet, daß** das selbe Stechelement (11) für den Hautöffnungsschritt und den Probengewinnungsschritt verwendet wird, wobei es am Ende des Hautöffnungsschritts abgebremst, vorzugsweise abgestoppt, und in dem Probengewinnungsschritt erneut beschleunigt wird.

5. Verfahren nach Anspruch 4, **dadurch gekennzeichnet, daß** das Stechelement (11) nach dem Abstoppen zurückgezogen, vorzugsweise vollständig aus der Epidermis (20) herausgezogen, wird.

6. Verfahren nach einem der vorhergehenden Ansprüche, **dadurch gekennzeichnet, daß** das Stechelement (11) vor dem Hautöffnungsschritt an die Hautoberfläche (20) herangefahren wird, mittels einer elektrischen Messung, die Lage der Hautoberfläche (20) an der Einstichstelle in Relation zu einem festen Referenzpunkt des verwendeten Geräts bestimmt wird.

7. Verfahren nach Anspruch 6, **dadurch gekennzeichnet, daß** das Stechelement (11) bei der elektronischen Messung als Elektrode verwendet wird und danach das Stechelement (11) in eine Ausgangsposition für den Hautöffnungsschritt zurückgezogen wird.

8. Verfahren nach einem der vorhergehenden Ansprüche,
**dadurch gekennzeichnet, daß**
das Stechelement (11) in dem Probengewinnungsschritt nach dem Vertiefen der Hautöffnung in eine Sammelposition zurückgezogen wird, in der sich seine Spitze noch in der Haut (20) befindet,
das Stechelement (11) bei Erreichen der Sammelposition abgebremst wird, und
anschließend seine Spitze während einer Sammelphase in der Haut (20) verbleibt, bevor sie vollständig aus der Haut (20) herausgezogen wird.

9. Verfahren nach einem der vorhergehenden Ansprüche, **dadurch gekennzeichnet, daß** die in dem Hautöffnungsschritt erzeugte Hautöffnung (21) vollständig in der Epidermis liegt und erst in dem Probengewinnungsschritt blutgebendes Gewebe erreicht wird.

10. Verfahren nach einem der vorhergehenden Ansprüche, **dadurch gekennzeichnet, daß** die bei dem Hautöffnungsschritt erzeugte Hautöffnung (21) eine Tiefe (A) von 0,4 mm bis 2,0 mm, bevorzugt mindestens 0,8 mm und höchstens 1,2 mm, hat.

11. Handgerät zum Erzeugen einer Einstichwunde, insbesondere ausgebildet zur Durchführung eines Verfahrens nach einem der vorhergehenden Ansprüche, umfassend ein Stechelement (11),
einen Antrieb (10), mit dem das Stechelement (11) in Richtung auf die Haut hin beweglich und in Richtung von der Haut weg beweglich ist, und
eine Steuereinrichtung (15) zum Steuern der Bewegung des Stechelementes (11),
**dadurch gekennzeichnet, daß**
es Mittel aufweist, durch die in einem Hautöffnungsschritt an einer Einstichstelle in der Epidermis (20) eine Hautöffnung (21) erzeugt wird, und
die Steuereinrichtung (15) so eingerichtet ist, daß nach dem Hautöffnungsschritt in einem Probengewinnungsschritt mittels des Stechelementes (11) ein Probengewinnungsstich ausgeführt wird, bei dem mit dem Stechelement (11) die Hautöffnung (21) vertieft und so eine Einstichwunde zum Gewinnen der Probe erzeugt wird.

12. Handgerät nach Anspruch 11, **dadurch gekennzeichnet, daß** die Mittel zum Erzeugen der Hautöffnung (21) einen Laser umfassen.

13. Handgerät nach Anspruch 11 oder 12, **dadurch gekennzeichnet, daß** die Steuereinrichtung so eingerichtet ist, daß das selbe Stechelement (11) für den Hautöffnungsschritt und den Probengewinnungsschritt verwendet wird, wobei es am Ende des Hautöffnungsschritts von der Steuereinrichtung (15) abgebremst, vorzugsweise abgestoppt, und in dem Probengewinnungsschritt erneut beschleunigt wird.

14. Handgerät nach einem der Ansprüche 11 bis 13, **dadurch gekennzeichnet, daß** der Antrieb eine Antriebsfeder zum Erzeugen einer Antriebskraft und einen Antriebsrotor, der unter Einwirkung der Antriebskraft eine Drehbewegung ausführt, umfaßt,
die Steuereinrichtung eine mit dem Antriebsrotor gekoppelte Kurvensteuerung umfaßt, durch die Drehbewegungen des Antriebsrotors in Bewegungen des Stechelementes (11) in Richtung auf die Haut hin und in Richtung von der Haut weg umgesetzt werden.

15. Handgerät nach Anspruch 14, **dadurch gekennzeichnet, daß** die Kurvensteuerung einen Steuerkurvenreiter aufweist, der bei einer Drehbewegung des Antriebsrotors eine Steuerkurve abfährt.

16. Handgerät nach Anspruch 15, **dadurch gekennzeichnet, daß** die Steuerkurve einen Verlauf hat, der ein erstes Extremum zum Erzeugen der Hautöffnung (21) und ein zweites Extremum zum Erzeugen der Einstichwunde aufweist.

17. Handgerät nach Anspruch 16, **dadurch gekennzeichnet, daß** mit dem zweiten Extremum der Steuerkurve ein mindestens ebenso großer Hub des Stechelementes (11) wie mit dem ersten Extremum der Steuerkurve verbunden ist.

18. Handgerät nach einem der Ansprüche 14 bis 17, **dadurch gekennzeichnet, daß** die Steuerkurve einen Drehwinkel des Antriebsrotors von mindestens 360° umfasst.

19. Handgerät nach Anspruch 18, **dadurch gekennzeichnet, daß** durch Entspannen der Antriebsfeder eine Drehbewegung des Antriebsrotors von mehr als 360°, vorzugsweise mindestens 540°, bewirkt wird und dabei der Steuerkurvenreiter einen Drehwinkel der Steuerkurve von mehr als 360°, vorzugsweise mindestens 540°, abfährt.

20. Handgerät nach einem der Ansprüche 15 bis 19, **dadurch gekennzeichnet, daß** die Steuerkurve eine offene Steuerkurve ist.

21. Handgerät nach einem der Ansprüche 14 bis 20, **dadurch gekennzeichnet, daß** sich der Antriebsrotor beim Entspannen der Antriebsfeder in einer ersten Drehrichtung dreht und der Antriebsrotor zum Spannen der Antriebsfeder in einer zweiten Drehrichtung gedreht wird, die der ersten Drehrichtung entgegengesetzt ist.

22. Handgerät nach einem der vorhergehenden Ansprüche 11 bis 21, **dadurch gekennzeichnet, daß** es eine Gehäuseöffnung (3) mit einem Andruckring (6) zum Anlegen eines Körperteils, in dem eine Einstichwunde erzeugt werden soll, aufweist.

## Geänderte Patentansprüche

### Geänderte Patentansprüche gemäss Regel 86(2) EPÜ.

**1.** Handgerät zum Erzeugen einer Einstichwunde, umfassend ein Stechelement (11),
einen Antrieb (10), mit dem das Stechelement (11) in Richtung auf die Haut hin beweglich und in Richtung von der Haut weg beweglich ist,
eine Steuereinrichtung (15) zum Steuern der Bewegung des Stechelementes (11), und
Mittel, durch die in einem Hautöffnungsschritt an einer Einstichstelle in der Epidermis (20) eine Hautöffnung (21) erzeugt und nach dem Hautöffnungsschritt in einem Probengewinnungsschritt eine Einstichwunde zum Gewinnen der Probe erzeugt wird
**dadurch gekennzeichnet, dass** es so ausgebildet ist, dass
das selbe Stechelement (11) für den Hautöffnungsschritt und den Probengewinnungsschritt verwendet wird, und
das Stechelement (11) am Ende des Hautöffnungsschritts von der Steuereinrichtung (15) abgebremst, vorzugsweise abgestoppt, und in dem Probengewinnungsschritt erneut beschleunigt wird, wobei die Hautöffnung (21) vertieft wird.

**2.** Handgerät nach Anspruch 1, **dadurch gekennzeichnet, daß** es so ausgebildet ist, daß zwischen dem Ende des Hautöffnungsschritts und dem Beginn des Probengewinnungsschritts ein zeitlicher Abstand von 1 msec bis 1 sec, vorzugsweise 1 msec bis 30 msec, liegt.

**3.** Handgerät nach einem der vorhergehenden Ansprüche, **dadurch gekennzeichnet, daß** es so ausgebildet ist, daß das Stechelement (11) nach dem Abstoppen zurückgezogen, vorzugsweise vollständig aus der Epidermis (20) herausgezogen, wird.

**4.** Handgerät nach einem der vorhergehenden Ansprüche, **dadurch gekennzeichnet, daß** es so ausgebildet ist, daß das Stechelement (11) vor dem Hautöffnungsschritt an die Hautoberfläche (20) herangefahren und mittels einer elektrischen Messung die Lage der Hautoberfläche (20) an der Einstichstelle in Relation zu einem festen Referenzpunkt des Geräts bestimmt wird.

**5.** Handgerät nach Anspruch 4, **dadurch gekennzeichnet, daß** es so ausgebildet ist, daß das Stechelement (11) bei der elektrischen Messung als Elektrode verwendet und danach in eine Ausgangsposition für den Hautöffnungsschritt zurückgezogen wird.

**6.** Handgerät nach einem der vorhergehenden Ansprüche, **dadurch gekennzeichnet, daß** es so ausgebildet ist, daß
das Stechelement (11) in dem Probengewinnungsschritt nach dem Vertiefen der Hautöffnung in eine Sammelposition zurückgezogen wird, in der sich seine Spitze noch in der Haut (20) befindet,
das Stechelement (11) bei Erreichen der Sammelposition abgebremst wird, und
anschließend seine Spitze während einer Sammelphase in der Haut (20) verbleibt, bevor sie vollständig aus der Haut (20) herausgezogen wird.

**7.** Handgerät nach einem der vorhergehenden Ansprüche, **dadurch gekennzeichnet, daß** es so ausgebildet ist, daß die in dem Hautöffnungsschritt erzeugte Hautöffnung (21) vollständig in der Epidermis liegt und erst in dem Probengewinnungsschritt blutgebendes Gewebe erreicht wird.

**8.** Handgerät nach einem der vorhergehenden Ansprüche, **dadurch gekennzeichnet, daß** es so ausgebildet ist, daß die bei dem Hautöffnungsschritt erzeugte Hautöffnung (21) eine Tiefe (A) von 0,4 mm bis 2,0 mm, bevorzugt mindestens 0,8 mm und höchstens 1,2 mm, hat.

**9.** Handgerät nach einem der vorhergehenden Ansprüche, **dadurch gekennzeichnet, daß** der Antrieb eine Antriebsfeder zum Erzeugen einer Antriebskraft und einen Antriebsrotor, der unter Einwirkung der Antriebskraft eine Drehbewegung ausführt, umfaßt; und die Steuereinrichtung eine mit dem Antriebsrotor gekoppelte Kurvensteuerung umfaßt, durch die Drehbewegungen des Antriebsrotors in Bewegungen des Stechelementes (11) in Richtung auf die Haut hin und in Richtung von der Haut weg umgesetzt werden.

**10.** Handgerät nach Anspruch 9, **dadurch gekennzeichnet, daß** die Kurvensteuerung einen Steuerkurvenreiter aufweist, der bei einer Drehbewegung des Antriebsrotors eine Steuerkurve abfährt.

**11.** Handgerät nach Anspruch 10, **dadurch gekennzeichnet, daß** die Steuerkurve einen Verlauf hat, der ein erstes Extremum zum Erzeugen der Hautöffnung (21) und ein zweites Extremum zum Erzeugen der Einstichwunde aufweist.

**12.** Handgerät nach Anspruch 11, **dadurch gekennzeichnet, daß** mit dem zweiten Extremum der Steuerkurve ein mindestens ebenso großer Hub des Stechelementes (11) wie mit dem ersten Extremum der Steuerkurve verbunden ist.

**13.** Handgerät nach einem der Ansprüche 10 bis 12, **dadurch gekennzeichnet, daß** die Steuerkurve einen Drehwinkel des Antriebsrotors von mindestens 360° umfasst.

**14.** Handgerät nach Anspruch 13, **dadurch gekennzeichnet, daß** durch Entspannen der Antriebsfeder eine Drehbewegung des Antriebsrotors von mehr als 360°, vorzugsweise mindestens 540°, bewirkt wird und dabei der Steuerkurvenreiter einen Drehwinkel der Steuerkurve von mehr als 360°, vorzugsweise mindestens 540°, abfährt.

**15.** Handgerät nach einem der Ansprüche 10 bis 14, **dadurch gekennzeichnet, daß** die Steuerkurve eine offene Steuerkurve ist.

**16.** Handgerät nach einem der Ansprüche 9 bis 15, **dadurch gekennzeichnet, daß** sich der Antriebsrotor beim Entspannen der Antriebsfeder in einer ersten Drehrichtung dreht und der Antriebsrotor zum Spannen der Antriebsfeder in einer zweiten Drehrichtung gedreht wird, die der ersten Drehrichtung entgegengesetzt ist.

**17.** Handgerät nach einem der vorhergehenden Ansprüche, **dadurch gekennzeichnet, daß** es eine Gehäuseöffnung (3) mit einem Andruckring (6) zum Anlegen eines Körperteils, in dem eine Einstichwunde erzeugt werden soll, aufweist.
